# EUROPEAN PATENT SPECIFICATION

(11) **EP 0 546 726 B1**
(45) Date of publication and mention of the grant of the patent: **25.09.1996**
(21) Application number: 92310843.5
(22) Date of filing: 27.11.1992
(51) Int. Cl.: A61F 2/38

(54) **Prosthetic knee joint**
Kniegelenksprothese
Prothèse d'articulation du genou

(30) Priority: 28.11.1991 GB 9125311
(43) Date of publication of application: 16.06.1993
(73) Proprietor: BIOMET LIMITED, Bridgend South Glamorgan CF31 3YN (GB)
(72) Inventor: Truscott, Jim, Swindon (GB)
(74) Representative: Yeadon, Mary Elizabeth

(56) References cited:
- EP-A- 0 183 670
- EP-A- 0 186 471
- EP-A- 0 498 586
- WO-A-89/06947
- WO-A-92/08424
- FR-A- 2 290 883

## Description

Prosthetic knee joints for the partial or complete replacement of the natural knee joint have been surgically used for some time.

The natural knee joint has inner and outer condyles or compartments and two bearings known as menisci. Known prosthesis may be adapted for the replacement of both condyles or only one condyle. Unicompartmental knee joint prosthesis are adapted to replace a single condylar joint, whereas bicompartmental knee joint prosthesis are adapted for the replacement of both condyles.

From British Patent No. 1534263 there is known a so-called meniscal knee joint. Meniscal knee joints comprise three components; a femoral component, a tibial component, and a meniscal component. In use the femoral component is attached to the patient's femur and the tibial component is attached to the patient's tibia. The meniscal component is placed between the femoral and tibial component and acts as a bearing surface. The meniscal component is free to slide with respect to the tibial and femoral components as the knee joint moves from flexion to extension. Rotational movement is also possible with a meniscal knee similar to that which occurs in a natural knee joint. One embodiment disclosed in British Patent No. 1534263 is a unicompartmental meniscal knee joint, in which the movement of the meniscal component with respect to the tibial component is limited by means of a under-cut recess in the meniscal component in which a projection from the tibial component is received. The extent of movement of the meniscal component is limited to the extent of movement of the projection within the recess.

From EP-A-0186471 there is known a meniscal knee joint which also incorporates such a feature. In this case the projection takes the form of a pin separably connected to the tibial component. A variety of complementary shapes are proposed for the end of the pin and the recess into which that end is accommodated. Those proposed shapes include circular, elliptical, star-shaped, square, slot-like and substantially triangular cross-sections.

From WO89/06947 there is known a different type of prosthetic knee joint which has a patellar component and a tibial component as well as a femoral component which has bearing surfaces for both the patellar and tibial components. The tibial component is formed of two parts; a tibial sleeve which is attached to the tibial bone and a tibial plateau component onto which the femoral component bears. The tibial components are fitted together by means of a tapering, downwardly extending shaft provided on the tibial plateau component and a correspondingly shaped aperture in the tibial sleeve into which the shaft is received. Translational movement between the two tibial components is prevented, i.e. the tibial plateau component does not slide with respect to the tibial sleeve. Relative rotation between the two components is allowed as the circular cross-sectional shaft of the tibial plateau component rotates within the circular cross-sectioned aperture in the tibial sleeve.

Although satisfactory prosthetic meniscal knee joints are on the market, there is always the need for improved prosthesis.

According to the present invention there is provided a prosthetic knee joint which comprises:
a bicompartmental femoral component capable of attachment to a femur and having a lateral and a medial articular bearing surface;
a bicompartmental tibial component capable of attachment to a tibia and having a medial and a lateral articular bearing surface; and
a bicompartmental meniscal component capable of acting as a bearing positioned between the bearing surfaces of the femoral and tibial components,
characterized in that the tibial component is provided between its medial and lateral articular bearing surfaces with a protruding member having at least at its distal end a cross-sectional shape which has a parallel-sided intermediate region extending between two convexly arcuate end regions, and in that
the meniscal component is provided with a recess in which the protruding member of the tibial component can be accommodated with play.

The recess of the meniscal component preferably has a cross-sectional shape which has an intermediate portion defined between two concavely arcuate sides which intermediate portion extends between two convexly arcuate end regions. Such shaping of the recess with respect to the protruding member is desired as it enables self-centring of the protruding member if it strikes the walls of the recess at an angle.

The recess of the meniscal component is preferably shaped so as to allow for longitudinal movement (i.e. in an anterior/posterior direction), lateral movement (i.e. in a medial/lateral direction), and rotational movement of the protruding member. Thus the meniscal component is preferably able to move in a lateral, longitudinal and rotational direction with respect to the tibial component.

The protruding member of the tibial component may be substantially capsule-shaped.

The recess of the meniscal component may be substantially peanut-shaped.

The degree of play possible in each of the above mentioned directions may not be equal. Where, for example, the recess has a cross-section having an intermediate portion defined by two concavely arcuate sides the degree of lateral play is less than that of the longitudinal play.

For both the protruding member and the recess the radius of curvature at their respective end regions may not be constant. The end regions of the recess could, for example, be shaped as part of an oval in that a central portion of the end region may have a greater radius of curvature than the outer portions of the end region. It is preferred that the radius of curvature at the extremity of the end regions of the recess is greater, or more preferably substantially equal to, that of the extremity of the end regions of the protruding member.

It is preferred that the cross-section of the protruding member has a longitudinal line of symmetry and/or a lateral line of symmetry. It is also preferred that the cross-section of the recess has a longitudinal line of symmetry and/or a lateral line of symmetry.

The protruding member and the recess are preferably of such relative proportions that the protruding member is allowed movement in the longitudinal direction of between 0.5 to 8mm, more preferably of 2 to 6mm and most preferably of around 4mm. It is also preferred that the relative dimensions of the protruding member and recess are such that the protruding member is allowed movement in the lateral direction of from 0.5 to 2mm, more preferably of 1 to 1.5mm, and most preferably of approximately 1.2mm. It is preferred that the relative dimensions of the recess and the protruding member are such that the protruding member is allowed movement in either rotational direction (i.e. either clockwise or anticlockwise) of 20°, more preferably of 15°, and most preferably of 12° of rotation as occurs in the natural knee joint.

The depth of recess is preferably less than or equal to the height of the protruding member. It is particularly preferred that the height of the protruding member is equal to the depth of the recess so that when the protruding member is received within the recess the meniscal component and the tibial component are in sliding contact.

The protruding member and recess may be so relatively shaped that engagement of the protruding member in the recess involves a snap action whereafter relative movement is possible.

The articular bearing surfaces of the tibial component are preferably both substantially planar in shape.

The medial and lateral articular bearing surfaces of the femoral component are also preferably planar in shape.

The meniscal component is preferably provided with bearing regions corresponding substantially in shape to the articular bearing surfaces with which they are in contact during use.

Where the medial and lateral articular bearing surfaces of the femoral component are substantially planar and correspond to substantially planar bearing surfaces on the meniscal component, the degree of contact between the femoral component and the meniscal component during use is great and is over an area of contact. In contrast, where the femoral articular bearing surfaces are arcuate in cross-section and the meniscal bearing surfaces have a substantially concavely spherical shape, due to the difficulty in exactly matching the shaping of the femoral and meniscal surfaces the contact may be reduced to either a line or a point of contact rather than a desired area of contact. In other words flat femoral articular bearing surfaces and corresponding flat bearing surfaces on the meniscal component lead to maximisation of congruence. Where the articular bearing surface of the femoral component is substantially planar the amount of natural articulation is increased (i.e. there is good congruence) and also wear is minimised.

The tibial component preferably takes the form of a planar plate from which the protruding member extends on one side and which is provided on its other side with means to assist its fixation to the proximal end of a tibia.

The tibial component in plan is preferably substantially kidney-shaped.

The meniscal component in plan is also preferably kidney-shaped.

The femoral component preferably takes the form of a laterally extending support from which two articulation members extend. The articulation members are each provided with a fixation surface and an articulation surface. The articular bearing surfaces are each provided on the articulation surface of one of the articulation members. The articulation surface is preferably convexly arcuate, but not of constant radius of curvature. The articulation surface preferably has a single radius of curvature extending from its anterior face through to an angle of from 20 to 50° from the longitudinal axis of the femur on which it is to be attached, more preferably through to an angle of between 30 to 40° and most preferably through to an angle of approximately 35° to that longitudinal axis. The radius of curvature anterior to the above mentioned single radius of curvature is preferably smaller. The convexly curved articulation surface may be formed by more than two curves of differing radius of curvature.

The shaping of the fixation surface of the femoral component may take any conventional form.

The femoral component is preferably anatomically shaped in that it is adapted to suit the anatomy of the patient.

Three conventional types of prosthetic knee joints are known. The first are constrained knee joints in which forward and backward movement only is allowed. The second are semi-constrained prosthesis which allow for forward and backward movement as well as for a very slight degree of rotation. The third are unconstrained prostheses which allow for forward and backward movement as well as rotational movement matching that provided naturally.

The prosthetic knee joint of the present invention is an unconstrained prosthesis. If during use fibrous tissue enters the recess of the meniscal component, leading to a reduction in the amount of play allowed for movement of the protruding member within the recess, the prosthesis will act as a semi-restrained or ultimately as a restrained prosthesis.

The shaping of the protruding member of the present invention is advantageous over the known use of circular protruding members as the area of contact between the protruding member and recess walls is greater leading to a greater distribution of the load/shock on the prosthesis as a whole.

For versatility of use, the meniscal component is preferably manufactured in a variety of sizes varying in thickness, for example varying in steps of 1mm.

The meniscal component is preferably made of an ultra-high molecular weight polyethylene (UHMWPE).

The tibial and femoral components are preferably made of a cobalt-chromium material, which has good wear resistance characteristics.

The metallic components may be provided with a coating, for example a porous coating of a titanium alloy or a plasma spray porous coating. On their fixation surfaces the femoral and tibial components are preferably provided with a surface preparation such as that known as the Interlok™ finish. This finish, which is formed by a blast process, increases the surface area in contact with the bone and enables a stronger bond between the prosthesis and the bone to be formed.

The femoral and tibial component may be fixed to the femur and tibia respectively by any conventional means, for example by the use of a bone cement, e.g. an acrylic bone cement. They may also be provided with attachment projections to assist in that fixation.

According to a second aspect of the present invention there is provided a bicompartmental tibial component capable of attachment to a tibia and having a medial and a lateral articular bearing surface and being provided between its medial and lateral bearing surfaces with a protruding member characterized in that the protruding member has at least at its distal end a cross-sectional shape which has a parallel-sided intermediate region extending between two convexly arcuate end regions.

According to a third aspect of the present invention there is provided a bicompartmental meniscal component capable of acting as a bearing positioned between a tibial and a femoral prosthetic component, which meniscal component is provided with a recess characterized in that the recess has a cross-sectional shape which has an intermediate portion defined between two concavely arcuate sides which intermediate portion extends between two convexly arcuate end regions.

For a better understanding of the present invention, and to show how the same may be put into effect, reference will now be made for the purposes of illustration only to the accompanying drawings in which:
Figure 1 is a side elevational view of an embodiment of a femoral component for use according to the present invention;
Figure 2 is a rear elevational view of the femoral component of Figure 1;
Figure 3 is a plan view of a meniscal component for use in the present invention;
Figure 4 is an enlarged detail of the recess of the meniscal component of Figure 3;
Figure 5 is a cross-section along line A-A of Figure 3;
Figure 6 is a cross-section along line B-B of Figure 3;
Figure 7 is a plan view of a tibial component for use in the present invention;
Figure 8 is an enlarged view of the protruding member of the tibial component of Figure 7; and
Figure 9 is a cross-section along lines A-A of Figure 7.
Figure 10 is a cross-section through an assembled embodiment of a prosthetic knee according to the present invention;
Figure 11 is a front elevation view of the knee of Figure 10;
Figure 12 is a side elevation of the knee of Figure 10 in an extension position; and
Figure 13 is a side elevation of the knee of Figure 10 in a flexed position.

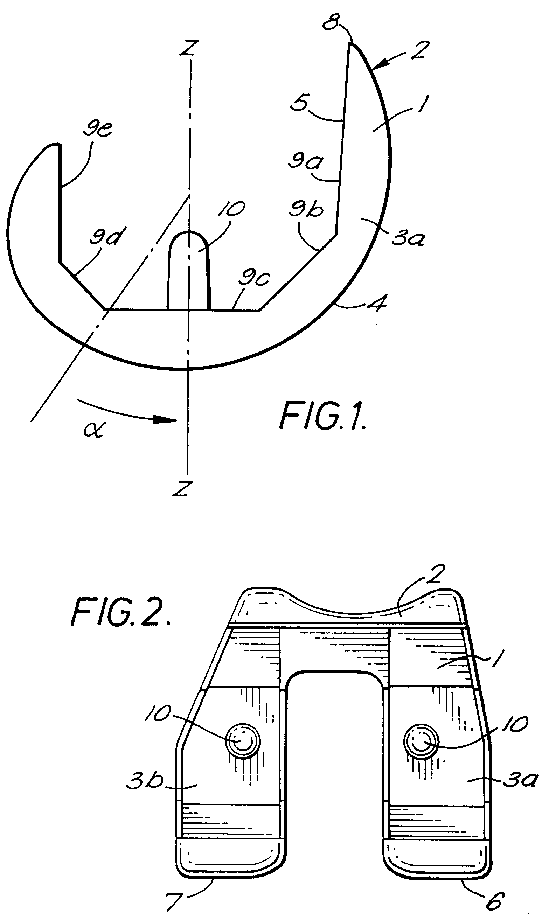

The femoral component 1 is anatomically shaped and has a support member 2 from which two articulation members 3a,b extend. Each articulation member 3a, 3b is provided with an articulation surface 4 and a fixation surface 5. The articulation surface is convexly arcuate in the longitudinal direction, but (as can be best seen in Figure 2) in the lateral direction is substantially planar, so that substantially planar lateral articular bearing surface 6 and medial articular bearing surface 7 are formed. The curve of the articulation surface 4 has a single radius of curvature from its anterior extremity 8 through to an angle α to the longitudinal axis line Z (which would be the longitudinal axis of the femur to which the femoral component 1 is attached in use). The remaining curvature of the articulation surface 4 is formed of curves of smaller radius of curvature. The fixation surface 5 is formed from a series of planar surfaces 9a,b,c,d,e and is provided with an attachment projection 10 extending upwards.

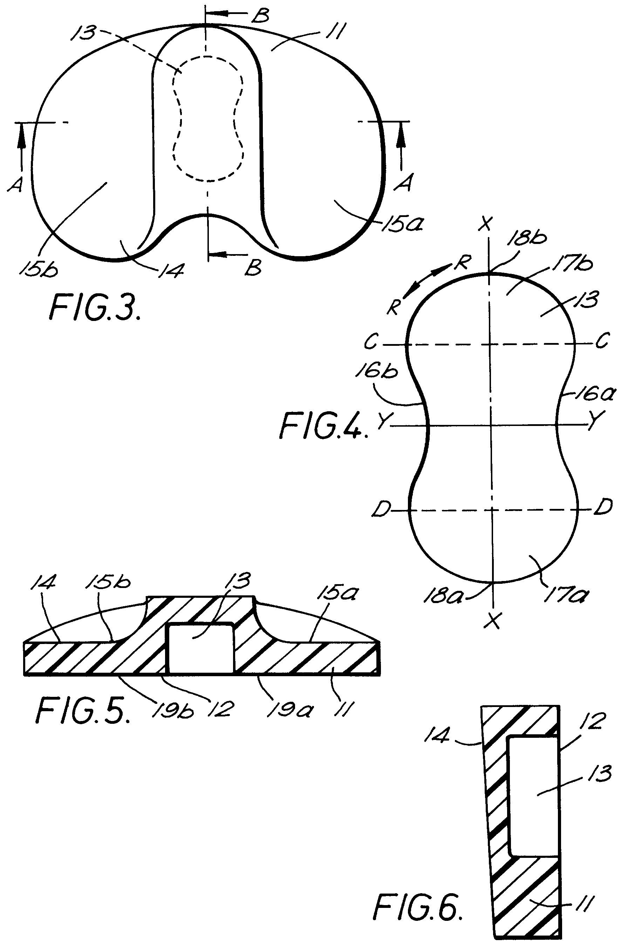

The meniscal component 11 is substantially kidney-shaped in plan view and is provided in its underside 12 with a substantially peanut-shaped recess 13. On its upper side 14 the meniscal component 11 has two substantially planar bearing surfaces 15a and b. The recess 13 in cross-section is symmetrical about the lines X and Y (see Figure 4). The recess in cross-section is formed from an intermediate region (defined between the lines C and D in Figure 4 which has concavely arcuate sides 16a and b extending between two convexly arcuate end regions 17a and b. The radius of curvature at the extremities 18a,b of the end regions 17a and b is greater than that towards the edges of the end regions 17a and b.

On the underside 12 of the meniscal component 11 is provided two flat tibial bearing surfaces 19a,b.

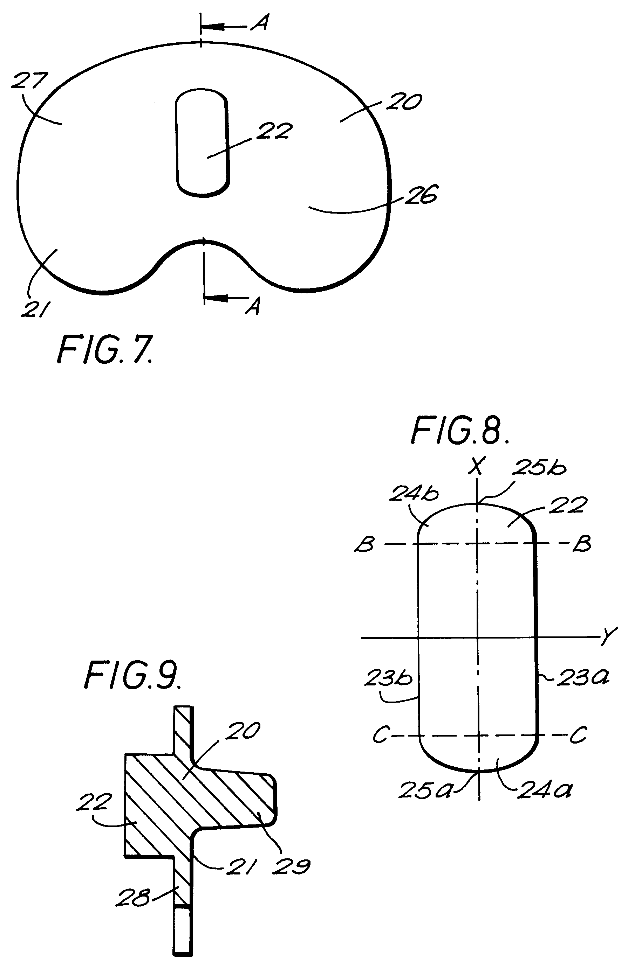

The tibial component 20 is in plan substantially kidney-shaped. It takes the form of a substantially planar plate from the upper surface 21 of which extends a protruding member 22. The protruding member 22 has a cross-sectional shape having an intermediate portion (defined between lines B and C of Figure 8) having parallel sides 23a,b which extend between convexly curved end regions 24a,b. The radius of curvature at the extremities 25a,b of the end regions 24a,b is equal to that at the extremities 18a,b of the recess 13.

The tibial component 20 is provided with a medial planar bearing surface 26 and a lateral planar bearing surface 27.

On the undersurface 28 of the tibial component 20 there is provided an attachment projection 29.

In use the femoral component 1 is fixed to the distal end of a femur and the tibial component 20 is fixed to the proximal end of a tibia. The meniscal component is positioned between the femoral component 1 and the tibial component 20 with the protruding member 22 of the tibial component 20 accommodated within the recess 13. Relative movement of the meniscal component 11 to the tibial component 20 is thereby allowed longitudinally (along the line X) laterally (along the line Y) and rotationally along the curve R (see Figure 4). The prosthetic knee joint is therefore able to mimic the amount of movement possible with a natural knee joint.

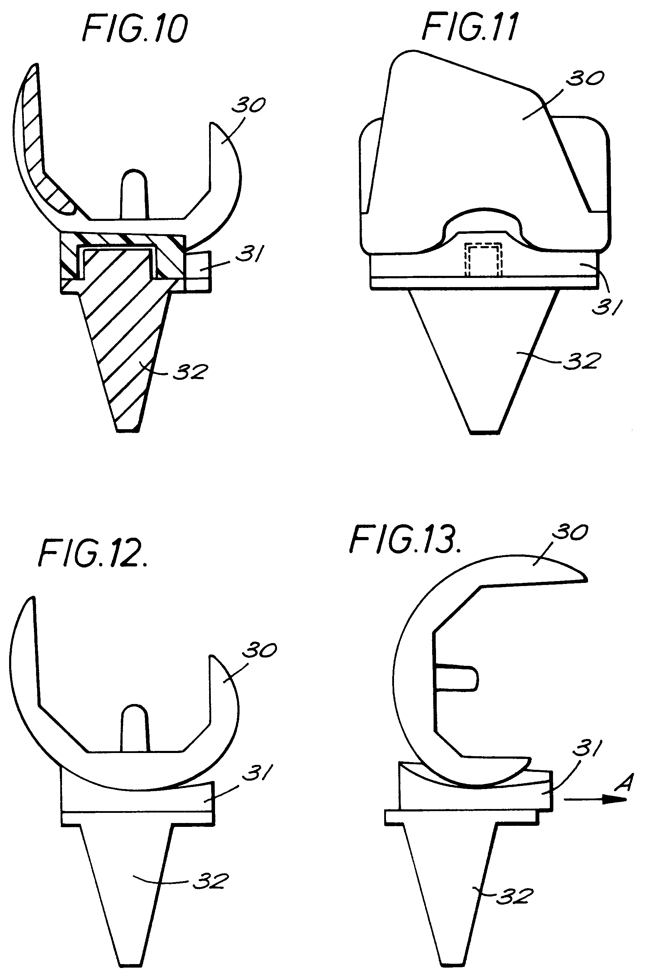

In figures 10 to 13 an assembled embodiment is shown. The femoral component 30, the meniscal component 31 and tibial component 32 are capable of relative movement. When the femur and tibia (not shown) of a patient in which the embodiment is fitted are fully extended the relative positioning of the components is shown in Figure 12. As the knee joint is flexed the femoral component 30 rolls over the meniscal component 31 which in turn slides in an anterior direction (along arrow A) relative to the tibial component 32 to the fully flexed position shown in Figure 13.

## Claims

1. A prosthetic knee joint which comprises:
a bicompartmental femoral component (1) capable of attachment to a femur and having a lateral (6) and a medial (7) articular bearing surface;
a bicompartmental tibial component (20) capable of attachment to a tibia and having a medial and a lateral articular bearing surface (26, 27); and
a bicompartmental meniscal component (11) capable of acting as a bearing positioned between the bearing surfaces of the femoral (1) and tibial (20) components,
characterised in that the tibial component (20) is provided between its medial and lateral articular bearing surfaces (26, 27) with a protruding member (22) having at least at its distal end a cross-sectional shape which has a parallel-sided (23a, b) intermediate region (B-C) extending between two convexly arcuate end regions (24a, b) and in that the meniscal component (11) is provided with a recess (13) in which the protruding member (22) of the tibial component (20) can be accommodated with play.

2. A prosthetic knee joint according to claim 1, wherein the recess (13) of the meniscal component (11) has a cross-sectional shape which has an intermediate portion (C-D) defined between two concavely arcuate sides (16a, b), which intermediate portion extends between two convexly arcuate end regions (17a, b).

3. A prosthetic knee joint according to claim 1 or 2, wherein the protruding member (22) of the tibial component (20) is substantially capsule-shaped.

4. A prosthetic knee joint according to claim 1, 2 or 3, wherein the recess (13) of the meniscal component (11) is substantially peanut-shaped.

5. A prosthetic knee joint according to any preceding claim, wherein the radius of curvature at the extremity of the end regions (17a, b) of the recess (13) is greater, or preferably substantially equal to, that of the extremity of the end regions (24a, b) of the protruding member (22).

6. A prosthetic knee joint according to any preceding claim wherein the medial and lateral articular bearing surfaces (13a, b) of the femoral component (1) are both planar in shape.

7. A prosthetic knee joint according to any preceding claim, wherein the femoral component (1) takes the form of a laterally extending support (2) from which two articulation members (3a, b) extend, each of which are provided with a fixation surface (15) and an articulation surface (4), the femoral articular bearing surfaces (6, 7) being each provided on the articulation surface of one of the articulation members, wherein the articulation surface is convexly arcuate, but not of constant radius of curvature and has a single radius of curvature extending from its anterior face through to an angle of from 20 to 50° from the longitudinal axis of the femur on which in use it is to be attached.

8. A bicompartmental tibial component (20) capable of attachment to a tibia and having a medial and a lateral articular bearing surface (26, 27) and being provided between its medial and lateral bearing surfaces with a protruding member (22), characterised in that the protruding member (22) has at least at its distal end a cross-sectional shape which has a parallel-sided (23a, b) intermediate region (B-C) extending between two convexly arcuate end regions (24a, b).

9. A bicompartmental meniscal component (11) capable of acting as a bearing positioned between a tibial and a femoral prosthetic component, which meniscal component (11) is provided with a recess (13) characterised in that the recess (13) has a cross-sectional shape which has an intermediate portion (C-D) defined between two concavely arcuate sides (16a, b) which intermediate portion extends between two convexly arcuate end regions (17a, b).

## Patentansprüche

1. Kniegelenksprothese, umfassend:
einen Femurbestandteil (1) mit zwei Kompartimenten, der an einem Femur befestigt werden kann und eine laterale (6) und eine mediale (7) Gelenkauflagefläche hat;
einen Tibiabestandteil (20) mit zwei Kompartimenten, der an einer Tibia befestigt werden kann und eine mediale und eine laterale Gelenkauflagefläche (26, 27) hat; und
einen Meniskusbestandteil (11) mit zwei Kompartimenten, der als Stütze wirken kann und sich zwischen den Auflageflächen der Femur- (1) und Tibiabestandteile (20) befindet,
dadurch gekennzeichnet, daß der Tibiabestandteil (20) zwischen seinen medialen und lateralen Gelenkauflageflächen (26, 27) mit einem vorstehenden Element (22) ausgestattet ist, das zumindest an seinem distalen Ende eine Querschnittsform hat, die einen zwischen zwei konvex gekrümmten Endbereichen (24a, b) verlaufenden parallelseitigen (23a, b) Zwischenbereich (B-C) besitzt,
und dadurch, daß der Meniskusbestandteil (11) mit einer Vertiefung (13) versehen ist, in der das vorstehende Element (22) des Tibiabestandteils (20) beweglich untergebracht werden kann.

2. Kniegelenksprothese nach Anspruch 1, bei der die Vertiefung (13) des Meniskusbestandteils (11) eine Querschnittsform hat mit einem Zwischenteil (C-D), der von zwei konkav gekrümmten Seiten (16a, b) definiert ist, wobei sich der Zwischenteil zwischen zwei konvex gekrümmten Endbereichen (17a, b) erstreckt.

3. Kniegelenksprothese nach Anspruch 1 oder 2, bei der das vorstehende Element (22) des Tibiabestandteile (20) im wesentlichen kapselförmig ist.

4. Kniegelenksprothese nach Anspruch 1, 2 oder 3, bei der die Vertiefung (13) dem Meniskusbestandteils (11) im wesentlichen erdnußförmig ist.

5. Kniegelenksprothese nach einem vorhergehenden Anspruch, wobei der Krümmungsradius am Ende der Endbereiche (17a, b) der Vertiefung (13) größer als oder vorzugweise im wesentlichen gleich dem des Endes der Endbereiche (24a, b) des vorstehenden Elementes (22) ist.

6. Kniegelenksprothese nach einem vorhergehenden Anspruch, wobei die medialen und lateralen Gelenkauflageflächen (13a, b) des Femurbestandteils (1) beide planar geformt sind.

7. Kniegelenksprothese nach einem vorhergehenden Anspruch, wobei der Femurbestandeil (1) die Form eines lateral verlaufenden Trägers (2) hat, aus dem die Gelenkelemente (3a, b) ragen, von denen jedes mit einei Befestigungsfläche (15) und einer Gelenkfläche (4) ausgerüstet ist, wobei sich die Femurgelenkauflageflächen (6, 7) jeweils auf den Gelenkflächen eines der Gelenkelemente befinden und wobei die Gelenkfläche konvex gekrümmt ist, jedoch keinen konstanten Krümmungsradius besitzt und einen einzigen Krümmungsradius hat, der sich von ihrer anterioren Seite in einem Winkel von 20 bis 50° von der Longitudinalachse des Femurs erstreckt, auf dem sie bei Gebrauch befestigt werden kann.

8. Tibiabestandteil (20) mit zwei Kompartimenten, der an eine Tibia befestigt werden kann und eine mediale und eine laterale Gelenkauflagefläche (26, 27) hat und zwischen seinen medialen und lateralen Auflageflächen mit einem vorstehenden Element (22) ausgestattet ist, dadurch gekennzeichnet, daß das vorstehende Element (22) zumindest an seinem distalen Ende eine Querschnittsform mit einem parallelseitigen (23a, b) Zwischenbereich (B-C) hat, der sich zwischen zwei konvex gekrümmten Endbereichen (24a, b) erstreckt.

9. Meniskusbestandteil (11) mit zwei Kompartimenten, der als zwischen einem Tibia- und einem Femurprothesebestandteil gelegene Stütze wirken kann, wobei der Meniskusbestandteil (11) mit einer Vertiefung (13) ausgerüstet ist, dadurch gekennzeichnet, daß die Vertiefung (13) eine Querschnittsform mit einem Zwischenteil (C-D) hat, der zwischen zwei konkav gekrümmten Seiten (16a, b) definiert ist, wobei sich der Zwischenbereich zwischen zwei konvex gekrümmten Endbereichen (17a, b) erstreckt.

## Revendications

1. Prothèse d'articulation de genou, qui comprend :
un composant fémoral (1) à deux compartiments, apte à être fixé à un fémur et possédant une surface de support articulaire latérale (6) et une surface de support articulaire médiale (7);
un composant (20) tibial à deux compartiments apte à être fixé à un tibia et possédant une surface de support articulaire médiale et une surface de support articulaire latérale (26,27); et
un composant en forme de ménisque (11) à deux compartiments, pouvant agir comme élément de support positionné entre les surfaces de support du composant fémoral (1) et du composant tibial (20),
caractérisée en ce que le composant tibial (20) comporte, entre ses surfaces de support articulaires médiale et latérale (26,27), un élément saillant (22) portant au moins, à son extrémité distale, une forme en coupe transversale qui possède une zone intermédiaire (B-C) à faces latérales parallèles (23a,b), qui s'étend entre deux parties d'extrémité courbes convexes (24a,b), et en ce que le composant en forme de ménisque (11) est pourvu d'un renfoncement (13), dans lequel l'élément saillant (22) du composant tibial (20) peut être logé avec jeu.

2. Prothèse d'articulation de genou selon la revendication 1, dans laquelle le renfoncement (13) du composant en forme de ménisque (11) possède une forme en coupe transversale qui comporte une zone intermédiaire (C-D) définie entre deux faces latérales courbes concaves (16a,b), laquelle partie intermédiaire s'étend entre deux parties d'extrémité courbes convexes (17a,b).

3. Prothèse d'articulation de genou selon la revendication 1 ou 2, dans laquelle l'élément saillant (22) du composant tibial (20) est essentiellement en forme de capsule.

4. Prothèse d'articulation de genou selon la revendication 1, 2 ou 3, dans laquelle le renfoncement (13) du composant en forme de ménisque (11) est essentiellement en forme de cacahuète.

5. Prothèse d'articulation de genou selon l'une quelconque des revendications précédentes, dans laquelle le rayon de courbure au niveau de l'extrémité des parties d'extrémité (17a,b) du renfoncement (13) est supérieur ou de préférence sensiblement égal à celui de l'extrémité des parties d'extrémité (17a,b) de l'élément saillant (22).

6. Prothèse d'articulation de genou selon l'une quelconque des revendications précédentes, dans laquelle les surfaces de support articulaires médiale et latérale (13a,b) du composant fémoral (1) possèdent toutes deux une forme plate.

7. Prothèse d'articulation de genou selon l'une quelconque des revendications précédentes, dans laquelle le composant fémoral (1) possède la forme d'un support (2) qui s'étend latéralement et à partir duquel s'étendent deux éléments d'articulation (3a,b), dont chacun est pourvu d'une surface de fixation (15) et une surface d'articulation (4), les surfaces de support articulaires fémorales (6,7) étant prévues chacune sur la surface d'articulation de l'un des éléments d'articulation, la surface d'articulation ayant une forme courbe convexe, mais sans posséder un rayon de courbure constant, et possédant un seul rayon de courbure qui s'étend depuis sa face antérieure sur un angle de 20 à 50° à partir de l'axe longitudinal du fémur, sur lequel il doit être fixé, en cours d'utilisation.

8. Composant tibial (20) à deux compartiments, apte à être fixé à un tibia possédant une surface de support articulaire médiale et une surface de support articulaire latérale (26,27) et comportant, entre ses surfaces de support médiale et latérale, un élément saillant (22), caractérisé en ce que l'élément saillant (22) comporte, au moins au niveau de son extrémité distale, une forme en coupe transversale qui comporte une région intermédiaire (B-C) pourvue de faces latérales (23a,b) et qui s'étend entre deux régions d'extrémité courbes convexes (24a,b).

9. Composant en forme de ménisque (11) à deux compartiments, apte à agir en tant que support disposé entre un composant tibial et un composant fémoral de prothèse, lequel composant en forme de ménisque (11) est pourvu d'un renfoncement (13), caractérisé en ce que le renfoncement (13) possède une forme en coupe transversale qui présente une partie intermédiaire (C-D) définie entre deux faces courbes concaves (16a,b), cette partie intermédiaire s'étendant entre les deux régions d'extrémité courbes convexes (17a,b).
